Europäisches Patentamt

European Patent Office

Office européen des brevets

(19)

(11) Publication number: **0 023 139**

A2

# (12) EUROPEAN PATENT APPLICATION

(21) Application number: **80302426.4**

(22) Date of filing: **18.07.80**

(51) Int. Cl.³: **A 61 B 10/00**
**A 61 B 17/32, A 61 B 1/00**

(30) Priority: **20.07.79 JP 92363/79**
**09.02.80 JP 15024/80**

(43) Date of publication of application:
**28.01.81 Bulletin 81/4**

(84) Designated Contracting States:
**BE CH DE FR GB IT LI NL SE**

(71) Applicant: **OLYMPUS OPTICAL CO., LTD.**
**43-2, 2-chome, Hatagaya Shibuya-ku**
**Tokyo(JP)**

(72) Inventor: **Omata, Katumi**
**551-5, Aihara**
**Sagamihara-Shi, Kanagawa-ken(JP)**

(72) Inventor: **Kato, Kiyoshi**
**6530-6, Hino**
**Hino-Shi, Tokyo(JP)**

(74) Representative: **Huntingford, David Ian et al,**
**W.P. THOMPSON & CO. Coopers Building Church Street**
**Liverpool L1 3AB(GB)**

(54) **Method of manufacturing a treatment instrument for an endoscope.**

(57) A treatment instrument for an endoscope includes members such as a cytodiagnostic brush assembly (10) or a forceps assembly (110), an operating wire (10a), and a flexible guide tube (81) which are held together by means of surrounding sleeves (32, 42, 81c). In order to minimize damage to the materials forming said members during fitting of the sleeve or sleeves (32, 42, 81c), the sleeves are applied by a drawing or thermo-compression bonding operation.

F I G. 5A

F I G. 5B

EP 0 023 139 A2

Croydon Printing Company Ltd

1.

## DESCRIPTION

"METHOD OF MANUFACTURING A TREATMENT INSTRUMENT FOR AN ENDOSCOPE"

The invention relates to a method of manufacturing various parts of a treatment instrument used with an endoscope, such as a cytodiagnostic brush, a forceps assembly, an operating wire, a flexible guide tube and the like.

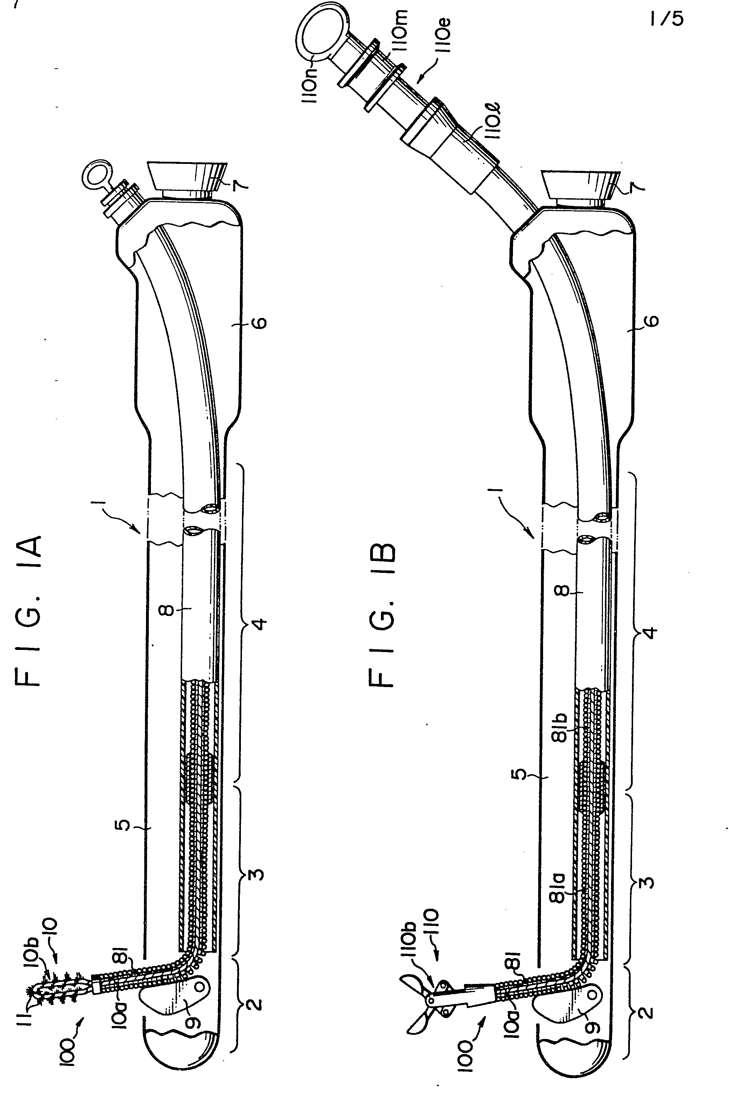

Referring to Figs. 1A and 1B of the accompanying drawings, there is shown a known endoscope 1 in which a treatment instrument 100 such as a cytodiagnostic brush (Fig. 1A) or forceps (Fig. 1B) is used by passing it through an insertion channel 8 formed in a portion 5 of the endoscope 1 which is adapted to be inserted into a coeliac cavity. The portion 5 has a distal end 2, a bendable portion 3 and a flexible portion 4. The portion 5 is formed by a flexible tubular member. In addition, the endoscope includes a proximate end operator 6 and an eyepiece assembly 7. A guide member 9 is disposed in the distal end 2 to direct the operative end of the treatment instrument. The treatment instrument 100 includes an operating wire 10a which extends through a resilient flexible guide tube 81 formed by a close pitch coil. The instrument 100 is detachably mounted in the endoscope 1 by passing the guide tube 81 into the insertion channel 8.

The treatment instrument 100 shown in Fig. 1A comprises a cytodiagnostic brush assembly 10 including a brush 10b which is mounted on the free end of the operating wire 10a and is located outside the resilient flexible tube 81 so as to be movable out of the distal end 2 into the coeliac cavity or back into the distal end 2.

The treatment instrument 100 shown in Fig. 1B comprises a forceps assembly 110 including forceps 110b

2.

mounted on the free end of the operating wire 10a and located outside the resilient flexible tube 81 so as to be movable out of the distal end 2 into the coeliac cavity for excising an affected part of the coeliac cavity such as polyp or for gripping such a part.

As is well known, the endoscope 1 is used for the purpose of observing and diagnosing an affected internal part of a coeliac cavity of a living person, such as the bronchus, stomach, colon or the like, by being inserted therein. While not shown, the distal end 2 of the endoscope is provided with an observation window and an illumination window. The bendable portion 3 is constructed as a pliable structure to facilitate the insertion of the endoscope portion 5 into the coeliac cavity for purpose of observation and diagnosis of an affected area, and the flexible portion 4 also is formed to be relatively pliable so as to be flexible in a resilient manner.

When the portion 5 of the endoscope 1 has been inserted into a coeliac cavity with its distal end 2 located opposite to an affected part, the cytodiagnostic brush assembly 10 is driven forward so that it projects out of the distal end 2 so as to be rubbed against the affected part in order to recover cell tissues thereof. The cytodiagnostic brush assembly 10 with recovered cell tissues is then withdrawn, together with the resilient flexible tube 81, through the channel 8 to be taken out of the endoscope.

In the case of Fig. 1B, when the endoscope portion 5 is inserted into the coeliac cavity with its distal end 2 located opposite to an affected part, the forceps 10b of the instrument 110 may be driven out of the distal end 2 and used to excise an affected part such as polyp or to recover cell tissues of the affected part.

3.

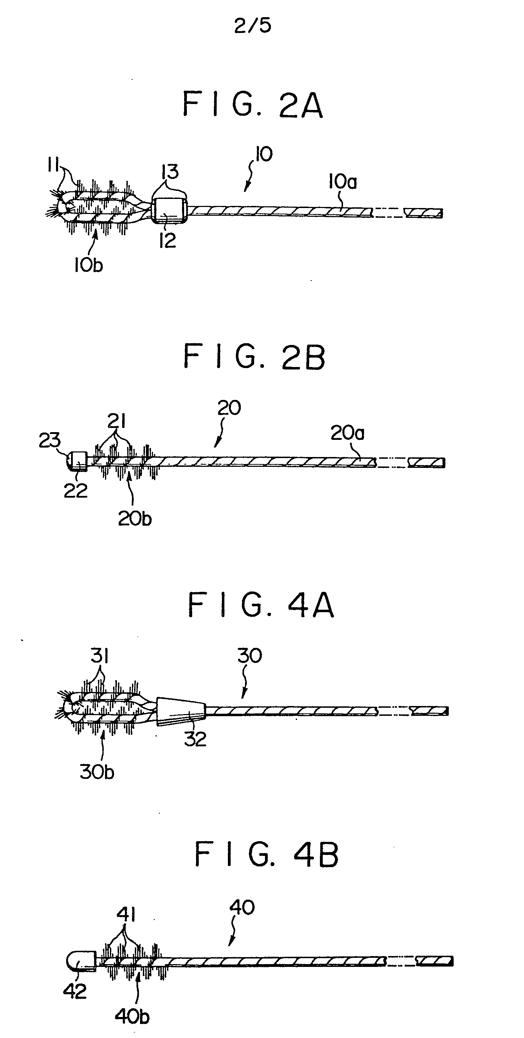

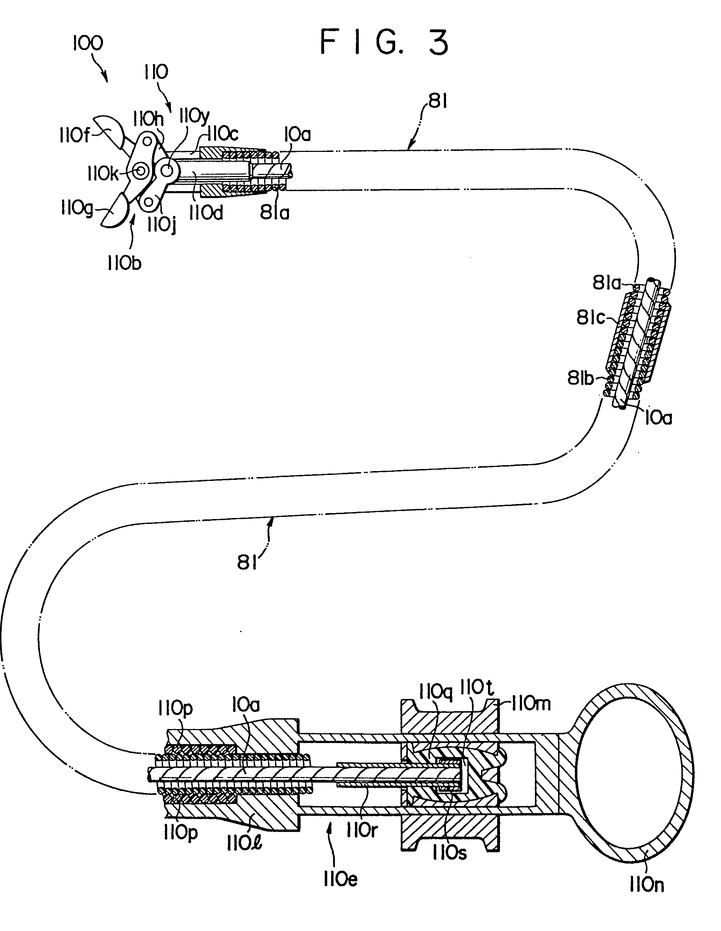

Fig. 2A shows more detail of the conventional construction of the cytodiagnostic brush assembly 10. As shown, it comprises the operating wire 10a which is formed by twisting a pair of resilient strands together, the brush 10b connected to the free end of the operating wire 10a and formed by the pair of resilient strands which are shaped into an oblong ring with a number of bristles 11, of a material such as nylon, held between the strands so as do form a generally oval-shaped scrubbing brush, and a short sleeve 12 which clamps the free ends of the strands forming the brush 10b together with the end of the operating wire 10a. Solder 13 is then injected into the clearance between the sleeve 12 and the operating wire 10a, between the sleeve 12 and the strands of the brush 10b, and between the operating wire 10a and the brush 10b. The solder 13 is shaped into a smooth profile to avoid the formation of any burr which may damage the internal wall of the coeliac cavity or the channel 8.

Fig. 2B shows another known cytodiagnostic brush assembly 20 including a brush 20b which is formed by a pair of initially straight resilient strands which are twisted together with bristles 21 held therebetween. A sleeve 22 is fitted over the free end of the strands of the brush 20b to cover and fix them, with solder 23 applied thereto and formed to present a smooth profile. The opposite end of the brush 20b is integrally formed with a straight operating wire 20a.

The cytodiagnostic brush assembly 10 or 20 thus formed may be used in the endoscope shown in Fig. 1A to project out of the flexible tube 81 so that the brush 10b or 20b may be used to recover cell tissues from an affected part of a coeliac cavity. The operating wire 10a or 20a of the cytodiagnostic brush assembly 10

4.

or 20 extends through the flexible tube 81 and through an outer tube 8. A forward most portion of the wire is pliably bent by the guide member 9. It is formed of a hard, resilient strand material so that it may be strongly pulled in order to pass it through the flexible tube 81 formed by a close pitch coil or to operate the brush 10b or 20b without causing a permanent deformation of the wire.

In the construction of the conventional cytodiagnostic brush assemblies 10, 20 shown in Figs. 2A and 2B, the sleeve 12 or 22 is fitted over the free end of the strands which define the brush, and solder applied thereto in order to cover and hold them in place. The end face of the solder must be formed or finished so as to present a smooth profile to prevent any damage from being caused to the internal wall of the coeliac cavity by the solder. Since the location where the solder is applied to the sleeve 12 or 22 is close to the bristles 11, 21, these bristles may be burnt as a result of heat from the soldering iron. A degree of skill and a significant working time are required to prevent the bristles from being burnt. To form a smooth profile of the solder applied, a special finishing operation must be performed, and the flux must be removed.

Fig. 3 shows in more detail the various members which constitute a treatment instrument. As shown, they include the forceps 110b, the operating wire 10a to the free end of which the rear end of a wire mounting stud 110d of the forceps 110b is secured, the resilient flexible tube 81 for guiding the axial movement of the operating wire 10a which extends therethrough, and a treatment instrument operator 110e which is connected to the rear end of the flexible tube 81 and the operating wire 10a. The forceps 110b includes a pair of forceps members 110f, 110g which are pivotally mounted

5.

on a pin 110k intermediate their ends and which are used to excise an affected part such as polyp or to hold it therebetween, and a pair of interconnecting arm members 110h, 110j which are pivotally mounted on a pin 110y and have their front ends articulated with the pair of forceps members 110f, 110g to open or close the latter members. The wire mounting stud 110d is in the form of a hollow shaft which is used to anchor the operating wire to its rear end, and fixedly carries the pin 110y on its front end. In addition, the forceps assembly includes a fixture 110c formed by a tubular member having the front end of the flexible tube 81 secured to its rear end and having the pin 110k fixedly mounted on its front end.

The resilient flexible guide tube 81 includes a coil pipe 81a of a relatively low hardness and having its front end secured to the fixture 110c of the forceps assembly 110b, another coil pipe 81b of relatively high hardness which is integrally joined to the rear end of the first mentioned coil pipe 81a, and a junction member 81c formed by a metal pipe which integrally connects the two coil pipes 81b, 81a together. A portion of the flexible tube which is formed by the coil pipe 81a corresponds to a length thereof which is located within the bendable portion 3 of the endoscope 1 as shown in Fig. 1B, and is pliable so as to be bendable. By contrast, another portion of the flexible tube formed by the coil pipe 81b represents a length thereof which is located within the flexible portion 4 of the endoscope 1 shown in Fig. 1B, and is constructed to permit a degree of resilient flexing.

The treatment instrument operator 110e comprises a flexible tube support member 110ℓ and an operating knob 110m. The rear end of the support member 110ℓ

6.

is formed with a finger ring 110n. By passing the thumb of an operator into the finger ring 110n and holding the knob 110m between the forefinger and the middle finger to operate it, the pair of forceps members 110f, 110g can be opened or closed by way of the operating wire 10a.

The support member 110$\ell$ has a front portion which is cylindrical in configuration, and a reinforcing coil pipe 110p of a reduced length is fitted around the inner cylindrical surface. The rear end of the coil pipe 81b is anchored inside the reinforcing coil pipe 110p. Intermediate the cylindrical front portion and the finger ring 110n, the support member 110$\ell$ is formed as a hollow cylinder provided with a pair of axially extending slits which divide the cross section of the support member into arcuate sections. The knob 110m is mounted on the slitted cylinder portion so as to be slidable therealong. An anchorage member 110q formed of a resilient material, e.g. a plastics material, is fitted into the central portion of the knob 110m, and the rear end of the operating wire 10a is fitted into the central opening in the anchorage member 110q and secured therein. In this manner, the operating wire 10a extends through the flexible tube 81, the front cylindrical portion of the support member 110$\ell$ and then extends into the knob 110m which is mounted on the slitted cylinder portion. An end protecting pipe 110r is integrally secured to the rear end of the wire 10a and its end integrally carries a stop 110s in the form of a short sleeve. It will be seen that the anchorage member 110q is fitted into a central opening in the knob 110m, which is shaped as shown to provide a firm bonding therewith.

In the prior art practice, the various members which constitute the treatment instrument and including the forceps 110b, the operating wire 10a, the flexible tube 81, the reinforcing coil pipe 110p, the end protecting pipe 110r and the stop 110s, are connected together by brazing or soldering. Specifically, the wire mounting stud 110d of the forceps assembly 110b is connected to the front end of the operating wire 10a by brazing while soldering is employed to connect the fixture 110c of the forceps assembly 110b with the front end of the coil pipe 81a, to connect the coil pipes 81a, 81b having different hardness by means of the junction member 81c, to connect the coil pipe 81b to the reinforcing coil pipe 110p, to connect the operating wire 10a to the end protecting pipe 110r, and to connect the pipe 110r to the stop 110s.

However, when such brazing or soldering techniques are used for the purpose of connection, the application of heat to parts which are to be connected together causes them to be partly annealed, whereby their mechanical strength is reduced. In addition, a number of post-processing steps are required including the removal of flux and subsequent drying. In addition, a degree of skill is required.

It is an object of the present invention to eliminate the above-described disadvantages of the prior art.

In accordance with the present invention, there is provided a method of manufacturing a cytodiagnostic brush for an endoscope having a sleeve holding together two elongate strands which, together with a plurality of bristles, form a brush characterized in that the sleeve of the cytodiagnostic brush is formed and secured in place by a drawing operation.

The invention also provides a method of manufacturing a treatment instrument for an endoscope which instrument includes members such as a cytodiagnostic

8.

brush or a forceps assembly, an operating wire, a flexible guide tube and the like which are connected together by means of a fastener member such as a sleeve, characterized in that a junction between the members such as the forceps assembly, the operating wire, the flexible guide tube and other associated members is achieved by forming the fastener member by a drawing or thermo-compression bonding operation applied to the peripheral part thereof.

The invention therefore avoids the use of soldering or brazing in order to interconnect members forming a treatment instrument for an endoscope, and hence the loss of strength of the members as a result of annealing them or the possibility that brush bristles may be burnt under heat used during the soldering or brazing operation is eliminated. In addition, a number of subsequent steps including the removal of flux and the subsequent drying operation are dispensed with. No skill is required as experienced when a soldering or brazing operation is used, and the working time can be greatly reduced. Since it is unnecessary to form the soldered or brazed parts into smooth profiles, a reduction in the manufacturing cost results.

The invention is described further hereinafter, by way of example, with reference to the accompanying drawings, in which:-

Figs. 1A and 1B are side elevations showing the important parts of two different, known treatment instruments mounted in an endoscope;

Figs. 2A and 2B are enlarged front views of conventional cytodiagnostic brushes of different designs;

Fig. 3 is an enlarged cross section of the treatment instrument shown in Fig. 1B;

Figs. 4A and 4B are enlarged front views of . different cytodiagnostic brushes formed by the method

9.

of the invention;

Figs. 5A and 5B are an enlarged cross section and a side elevation of drawing equipment which may be used to carry out the method of the invention;

Fig. 6 is an enlarged cross section of a sleeve which is secured in place by the method of the invention;

Fig. 7 is a side elevation of a forming equipment used in the method of the invention;

Figs. 8A and 8B are a front view and a side elevation of a sleeve which is secured to the cytodiagnostic brush with the equipment of Fig. 7;

Fig. 9A is an enlarged front view of a drawing equipment used in the method of the invention;

Fig. 9B is an enlarged cross section of a treatment instrument worked with the equipment of Fig. 9A;

Fig. 10 is an enlarged side elevation of a thermo-compression bonding apparatus used in the method of the invention; and

Figs. 11, 12 and 13 are enlarged cross sections of parts of a treatment instrument which are connected together with the apparatus shown in Fig. 9A or 10.

Referring to Figs. 4A and 4B, there are shown cytodiagnostic brush assemblies 30, 40 having sleeves 32, 42, which are applied thereto and formed in accordance with the method of the invention. In accordance with the invention, these sleeves 32, 42 are formed and secured by a drawing or thermo-compression bonding operation, thus dispensing with a soldering operation and accompanying finishing operations to achieve a smooth profile in order to prevent the wall of the coeliac cavity from being damaged. Hence, there is no possibility that the bristles 31, 41 of the brushes 30b, 40b may be burnt by heat from a soldering iron as experienced in the prior art. Simultaneously, the need for soldering skill is eliminated, and the working time can be substantially reduced. It is unnecessary to form the soldered portion into a smooth profile and this results in a reduced manufacturing cost.

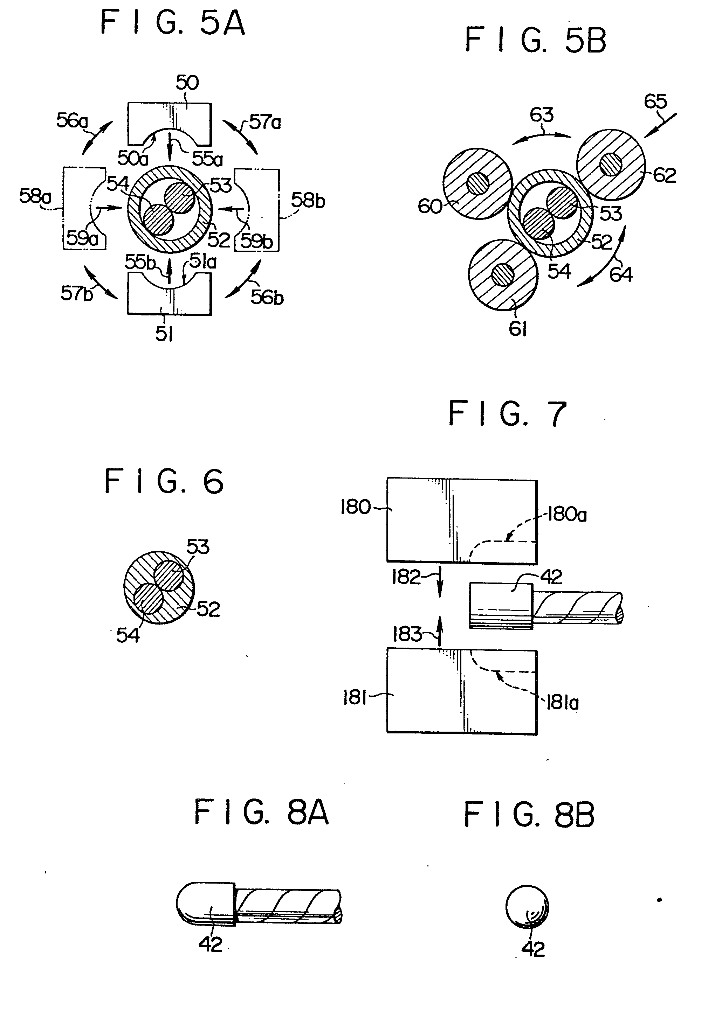

Figs. 5A and 5B illustrate a drawing apparatus which may be used to carry out the method of the invention. In Fig. 5A, a drawing apparatus which employs drawing dies to secure a sleeve in place is illustrated. Specifically, the apparatus includes a pair of vertically spaced dies 50, 51 which are formed with arcuate recesses 50a, 51a formed in their opposing surfaces. A sleeve 52 which is to be applied to the strands of the cytodiagnostic brush assembly is disposed between the dies 50, 51, with the free end of a strand 53 and the opposite end of another strand 54, both of which are used to form the brush 30b (Fig. 4A), disposed in juxtaposed relationship within the sleeve 52. The dies 50,51 are then driven in directions indicated by arrows 55a, 55b to stake the sleeve 52 by pressing it from opposite directions. The dies 50, 51 are then returned to their original positions, followed by a rotation thereof in directions indicated by arrows 56a, 56b or arrows 57a, 57b while maintaining the opposing relationship of the recesses 50a, 51a. In this manner, these dies are brought to a position shown in phantom lines 58a, 58b where they have been rotated through 90°, for example. The dies 50, 51 are then driven in directions indicated by arrows 59a, 59b to stake the sleeve 52 again by pressing it from the opposite lateral directions. By repeating such operation to press the sleeve 52 in vertical, lateral and oblique directions as viewed in Fig. 5 by means of the dies 50, 51, the sleeve 52 is staked by a drawing operation, with the consequence that the metal of the sleeve 52 is deformed into a configuration as shown in Fig. 6 where it will be seen that the metal flows into the space between the strands 53, 54 to cover and fix them. It should be understood that a plurality of dies which are greater in number than the pair of dies shown may be used for the purpose of the drawing operation. Also it should be understood that instead of rotating the dies 50, 51 as shown in the embodiment of Fig. 5A, the sleeve 52 may be angularly moved in relation to the dies 50,51.

Fig. 5B illustrates a drawing apparatus which utilizes the principle of operation of a pipe cutter. As before, the opposite ends of strands 53, 54 are inserted into a sleeve 52, which is disposed so that its left-hand side (as viewed in Fig. 5B) is disposed in abutment against a pair of fixed rollers 60, 61. Another, movable roller 62 is disposed to bear against the sleeve 52 on its upper, right-hand peripheral surface. The movable roller 62 is driven toward the center of sleeve 52, in a direction indicated by an arrow 65, while rotating or angularly moving in directions indicated by arrows 63, 64. In this manner, as the movable roller 62 rotates, the sleeve 52 is caused to rotate simultaneously, whereby its entire peripheral surface is uniformly pressed. At the same time, as the movable roller 62 is angularly displaced, the sleeve is again uniformly pressed from different directions. When the sleeve 52 is uniformly pressed in every direction, it is staked by a drawing operation in the same manner as by the use of the dies shown in Fig. 5A. As a result, the metal of the sleeve 52 flows into space between the strands 53, 54 to cover and fix them, as shown in Fig. 6.

12.

The drawing apparatus illustrated in Figs. 5A and 5B can be used to achieve staking of the sleeves 32, 42 of the cytodiagnostic brush assembly 30, 40 shown in Figs. 4A and 4B.

Referring to Fig. 7, there is shown a pair of dies 180, 181, in side elevation, which may be used to shape the sleeve 42 used to cover and fix the free end of the strands of the brush 40b shown in Fig. 4B. The sleeve 42 is to be secured to the free end of the brush with its front end shaped into a rounded form. Accordingly, the opposing surfaces of the dies 180, 181 are formed with recesses 180a, 181a, the front end of which is rounded. By placing the sleeve 42 between the recesses 180a, 181a of the dies and driving the dies 180, 181 in directions indicated by arrows 182, 183, the sleeve 42 can be staked into shape as shown in Figs. 8A and 8B and secured to the strands, with its forward end rounded. The sleeve 42 may be in the form of a hollow pipe or a blind pipe. The material for the sleeve principally comprises stainless steel.

The interconnection of the various members of the forceps assembly 110 shown in Fig. 3 including the forceps 110b, operating wire 10a, flexible tube 81, reinforcing coil pipe 110p, end protecting pipe 110r and stop 110s in accordance with the method of the invention will be described below.

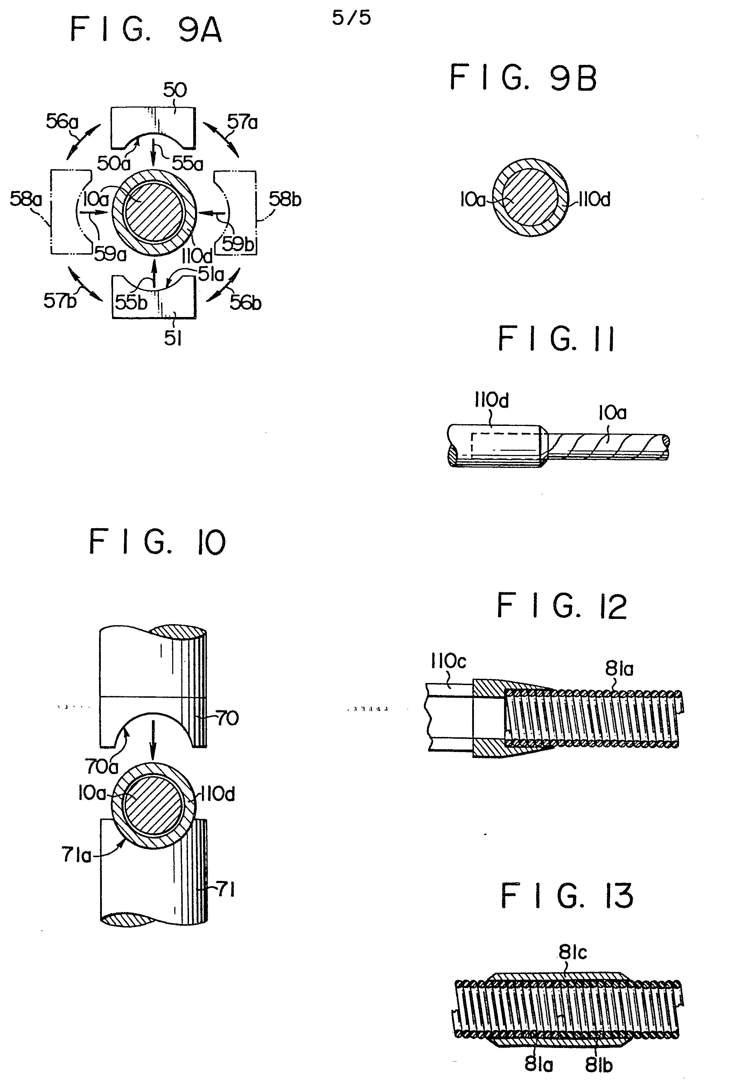

In Fig. 9A, a drawing operation which utilizes a pair of dies is employed to connect the wire mounting stud 110d and the wire 10a together (see Fig. 3). In this arrangement, a pair of dies 50, 51 which have arcuate recesses 50a, 51a formed in their opposing surfaces are vertically spaced apart. Disposed between the dies 50, 51 is the hollow rear portion of the wire mounting stud 110d, into which the front end of the wire 10a is inserted as indicated in Fig. 11. When the

13.

dies 50, 51 are subsequently driven in opposite directions indicated by arrows 55a, 55b to press the stud 10d therebetween, it is staked. The dies 50, 51 are then moved away from the stud 110d and returned to their original positions, and thereafter they are angularly moved in directions indicated by arrows 56a, 56b or arrows 57a, 57b while maintaining the opposing relationship between the recesses 50a, 51a. In this manner, they may be brought to suitable positions such as phantom line positions 58a, 58b which are displaced by 90° from their original positions. The dies 50, 51 are then driven in directions indicated by arrows 59a, 59b to press the stud 110d therebetween in the lateral direction, thus again staking it. By repeating such operation a number of times, the stud 110d is staked by a drawing operation as a result of pressing the stud 110d between the dies 50, 51 in vertical, lateral, oblique and other directions. The consequence is a flow of the metal which forms the stud 110d into firm bonding with the wire 10a, as shown in Fig. 8B, thus achieving an interconnection therebetween. It will be appreciated that instead of using a pair of dies, an increased number of dies may be used to achieve a similar effect. Also, it is to be understood that while in the embodiment shown in Fig. 9A, the drawing operation of the stud 110d has been performed by angularly moving the dies 50, 51 through angular increments, the stud 110d may be rotated instead.

Fig. 10 shows an arrangement which utilizes a spot welding technique to heat the stud 110d while compressing it. Specifically, a pair of spot welding electrodes 70, 71 are vertically spaced apart and have arcuate recesses 70a, 71a formed in their opposing surfaces which are of a diameter less than the outer diameter of the stud 110d. The front end of the operating wire 10a is inserted into the hollow rear portion of the stud 110d, which is then placed in the recess 71a of the lower electrode 71. Then, the upper electrode 70 is driven toward the stud 110d until its recess 70a is brought into abutment against the peripheral surface of the stud 110d, thus vertically pressing it between the electrodes 70, 71. By applying a voltage across the electrodes 70, 71, Joule heat is produced in the stud 110d by a current flow which passes therethrough, whereby the stud 110d is heated. When the electrodes 70, 71 are further driven under this condition to press the stud 110d therebetween, the latter can be easily deformed into the configuration of the arcuate recesses 70a, 71a. In this manner, the stud 110d is staked. Again, the metal of the stud 110d flows to cover and join with the wire 10a in an integral manner, as shown in Fig. 9B.

When the spot welding technique is employed, the parts joined together will be heated to a higher temperature than that which occurs when a conventional soldering or brazing technique is employed. However, the localized high temperature region which is more restricted than a corresponding region experienced with a brazing operation, for example, combined with a reduced length of working time prevents a loss of the mechanical strength as a result of an annealing, thus preserving the high strength which is inherently obtained with a spot welding technique. It should be noted nevertheless that because the members are welding together at spot locations, a number of spot welding operations must be repeated along the circumference in order to increase the overall strength.

15.

The described drawing operation or the thermo-compression bonding operation which utilizes spot welding avoids the need for post-processing steps such as the removal of flux or drying, thus enabling a reduction in the number of processing steps. In addition, an excellent finish is obtained, providing a good appearance. The reduced number of working steps and lack of necessary skill permit a reduction in the process cost.

Fig. 12 shows a junction between the fixture 110c of the forceps assembly 110b and the coil pipe 81a. Again, the drawing operation illustrated in Fig. 9A or the thermo-compression bonding illustrated in Fig. 10 may be utilized to connect these members together.

Fig. 13 illustrates the connection between the rear end of the coil pipe 81a and the front end of the coil pipe 81b by utilizing the intermediate connecting member 81c. In this instance, the drawing operation or the thermo-compression bonding illustrated in Figs. 9A and 10 may be applied to the intermediate member 80c, to connect these members together.

In forming the junctions illustrated in Figs. 12 and 13, the hollow construction of the parts to be connected together requires the insertion of a core of a corresponding diameter inside the associated parts before the operation illustrated in Figs. 9A and 10 takes place. Subsequently, the core may be removed, leaving both members which are integrally connected together. When the tubular portion of the fixture 110c or the intermediate connection member 80c is fitted over the coil pipe 81a, and the both members are integrally connected by a drawing operation, the external profile of the coil pipe may cause female threads to be formed in the internal surface of the tubular portion of the

16.

fixture 110c or the member 81c. This results in the likelihood of the both members being separated as they are turned relative to each other. However, such likelihood can be avoided by utilizing different pitches for the two coils, or by providing a flute in part thereof which prevents the separation.

It should be understood that the interconnecting method of the invention is also applicable to the connection between the coil pipe 81b and the reinforcing coil pipe 110p, the connection between the wire 10a and the end protecting pipe 110r, and the connection between the end protecting pipe 110r and the stop 110s, all shown in Fig. 2. Either a drawing operation or a thermo-compression bonding operation may be equally applied.

17.

CLAIMS

1. A method of manufacturing a cytodiagnostic brush for an endoscope having a sleeve (32,42) holding together two elongate strands which, together with a plurality of bristles, form a brush (30b,40b), characterized in that the sleeve (32,42) of the cytodiagnostic brush (30, 40) is formed and secured in place by a drawing operation.

2. A method according to claim 1 in which the step of forming the sleeve (32, 42) by a drawing operation employs a plurality of dies (50,51).

3. A method according to claim 1 in which the step of forming the sleeve (32, 42) by a drawing operation employs a pipe cutter mechanism including a plurality of stationary rollers (60, 61) and at least one movable roller (62).

4. A method of manufacturing a treatment instrument for an endoscope which instrument includes members such as a cytodiagnostic brush or a forceps assembly, an operating wire, a flexible guide tube and the like which are connected together by means of a fastener member such as a sleeve, characterised in that a junction between the members such as the forceps assembly (110), the operating wire (10a), the flexible guide tube (81) and other associated members is achieved by forming the fastener member (81c, 110c, 110d, 110p, 110r, 110s) by a drawing or thermo-compression bonding operation applied to the peripheral part thereof.

5. A method according to claim 4 in which the step of forming the fastener member by a drawing operation employs a plurality of dies (50, 51).

6. A method according to claim 4 in which the step of forming the fastener member by a thermo-compression bonding operation employs a pair of spot

18.

welding electrodes (70, 71) having arcuate recesses
(70a, 71a) in their opposing surfaces.

# FIG. IA

# FIG. IB

0023139

1/5

A 1211 F

# F I G. 2A

# F I G. 2B

# F I G. 4A

# F I G. 4B

A12111F

# F I G. 3

A12111F

# F I G. 5A

50
56a
57a
50a 55a
54 53
58a 58b
59a 52 59b
55b 51a
57b 56b
51

# F I G. 5B

63 65
60 62
53
52
54
61 64

# F I G. 7

180 180a
182
42
183
181 181a

# F I G. 6

53
52
54

# F I G. 8A

42

# F I G. 8B

42

FIG. 9A

FIG. 9B

FIG. 11

FIG. 10

FIG. 12

FIG. 13